# EUROPEAN PATENT APPLICATION

(11) **EP 1 887 013 A1**
(43) Date of publication of application: **13.02.2008**
(21) Application number: 05772872.7
(22) Date of filing: 18.07.2005
(51) Int. Cl.: C07H 19/04

(54) **A PROCESS FOR PREPARING DIDANOSINE**

(30) Priority: 30.05.2005 CN 200510026291
(71) Applicant: Shanghai Aurisco International Trading Co., Ltd., Shanghai 200063 (CN)
(72) Inventor: CHU, Dingjun, Zhejiang 317200 (CN); ZHANG, Defa, Zhejiang 317200 (CN)
(74) Representative: Korga, Leokadia
(86) International application number: PCT/CN2005/001059
(87) International publication number: WO 2006/128328

(57) **Abstract**

This invention provides a method for preparing didanosine. The method comprises removing a protecting group in position 5' of compound II by hydrolysis under basic reaction conditions, and simultaneously enolizing of the carbonyl group of the purine ring to obtain a stable salt, and then producing the salt of 2',3-dideoxyinosine through catalytic hydrogenation, which salt is finally converted with acid to yield the finished product.

## Description

Field of the Invention

The invention relates to a method for the preparation of didanosine.

Background of the Invention

Didanosine is an antiviral drug used to treat human immunodeficiency virus (HIV) infection. It was developed at the National Cancer Institute (US Pat No.: 4,861,759). The chemical name for didanosine is 2',3'-dideoxyinosine (DDI). Didanosine is one of the most important anti-HIV drugs. There are two main synthetic routes to didanosine: 1) catalytic hydrogenation of compound II (below) and removal of protecting group; and 2) removal of protecting group (R) in position 5' from compound II to obtaib 2',3'-didehydro-2',3'-dideoxyinosine ("D4l"), followed by hydrogenated to obtain DDI.

The two methods have the disadvantages of low yield and high cost. However, HIV-infected patients generally require inexpensive treatment, which limits the application of didanosine.

Description of the invention:

The problem to be solved is to overcome the shortcomings of the methods mentioned above, and to provide a new method for preparing 2',3'-dideoxyinosine (DDI) of a high purity.

Thus, the invention provides a new method to prepare didanosine of formula I.

This new method comprises (a) removing the protecting group in position 5' of the compound II by hydrolysis under basic reaction conditions, and simultaneously enolizing the carbonyl group of the purine ring to yield the stable salt III, i.e. the salt of 2',3'-didehydro-2',3'- dideoxyinosine (X-D4l); (b) producing the salt of 2',3'-dideoxyinosine(X-DDI) through catalytic hydrogenation of X-D4l; and (c) neutralizing X-DDI with acid to obtain compound I, i.e., didanosine.

The method is illustrated as follows: 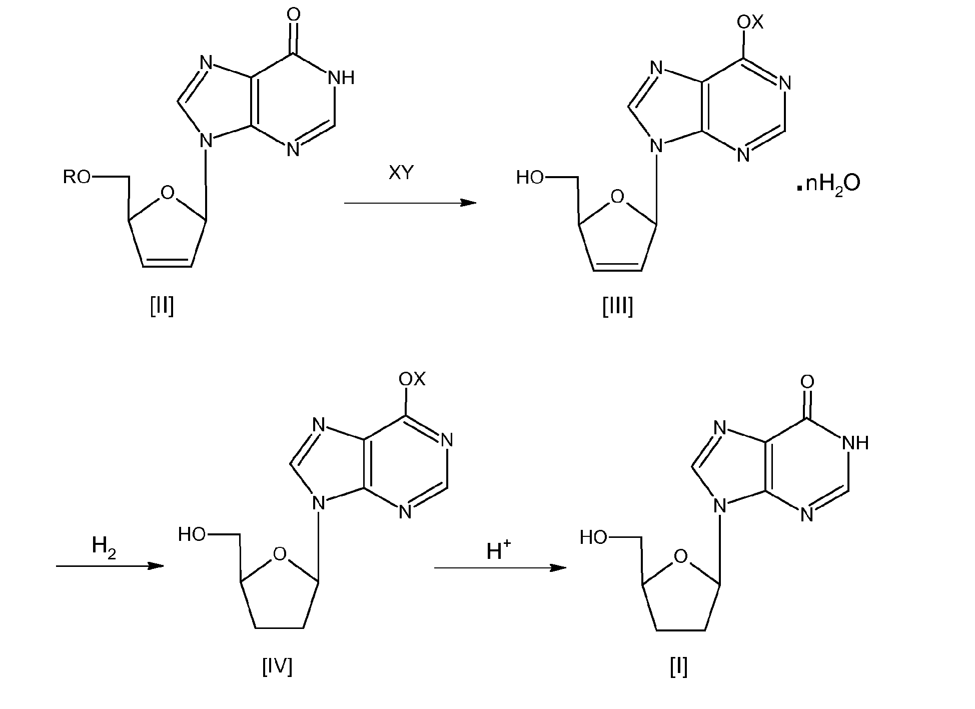 wherein X is a cation, including an organic cation, such as, e.g., an alkali metal ion, an ammonium ion, or a quaternary ammonium cation; preferably, Na⁺, K⁺, NH₄⁺ or CH₃NH₃⁺, and more preferably Na⁺; Y is a corresponding anion; R is acyl, acetoxy, alkyl, or silyl, such as, e.g., acetyloxy, 2,5,5-trimethyl-1,3-dioxolane -4-carboxylate, or tert-butyldimethylsilyl.

When pH value is maintained above 8, compound IV is obtained by catalytically hydrogenating compound III or its hydrate in a purified form or as crude solution from the previous step. The pH in the range of between 8.5 and 9.5 is preferable for hydrogenation. The catalyst used in hydrogenation is Raney nickel or palladium carbon, and it can be re-used. Then, compound IV is neutralized with acid to yield didanosine of high purity.

When compound III is hydrogenated after purification, a suitable solvent should be used and the pH should be maintained at above 8. The solvent can be water, ammonia water, lye, alcohol, alcohol water mixtures, ketone water mixtures, or nitrile water mixtures, etc., such as, e.g., methanol, a mixture of methanol and water, a mixture of ethanol and water, a mixture of isopropanol and water, a mixture of acetonitrile and water, a mixture of acetone and water, ammonia water, or a mixture of methylamine and water, etc.

The acid used for neutralization is an inorganic acid or an organic acid, such as, e.g., hydrochloric acid, acetic acid, etc, and preferably acetic acid.

The base used in the hydrolysis of compound II to compound III can be an inorganic base, an organic base, or a mixture thereof, as long as the pH of the solution is maintained above 8.5, and preferably the base is a solution of potassium hydroxide, sodium hydroxide, potassium carbonate, sodium carbonate, or methylamine.

The other object of the invention is to provide a new key intermediate compound III (X-D4l*·*nH₂O) useful in the synthesis of didanosine. Said compound is a water soluble, stable salt of 2',3'-didehydro-2',3'- dideoxyinosine, the structural formula of which is as follows: 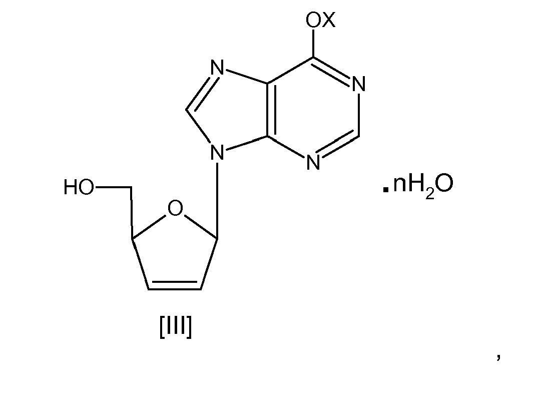 wherein X is a cation, such as, e.g., Na⁺, K⁺, Mg⁺⁺, Ca⁺⁺, or NH₄⁺, and n is between 0 and 3. These salts include salts obtained with organic cations, alkali, alkali metal ions, ammonium ions, or quaternary ammonium cations, and sodium salts are preferable. An alkali metal salt can be obtained by the reaction of an alkali metal with compound II. When the pH of the solution is above 8, the product is very stable. In solid form, the product is a hydrate and the number of water molecules is between 0.5 and 3.

Compound II is hydrolyzed in a solution with the pH above 8.5 to yield X-D4l. After adding organic solvent, compound III (X-D4l·nH₂O) crystallizes out. The base used in hydrolysis is an inorganic base, an organic base, or a mixture thereof; and preferably potassium hydroxide, sodium hydroxide, potassium carbonate, sodium carbonate, or methylamine. The organic solvent should be water-soluble, e.g., an alcoholic solvent, a ketonic solvent, a nitrilic solvent, etc., such as, e.g., isopropanol, acetone, acetonitrile, or tetrahydrofuran.

The method presented herein produces didanosine with high purity, high yield, high quality, and low cost.

The following examples are intended to show how to practice of invention and are not intended to restrict the scope of thereof.

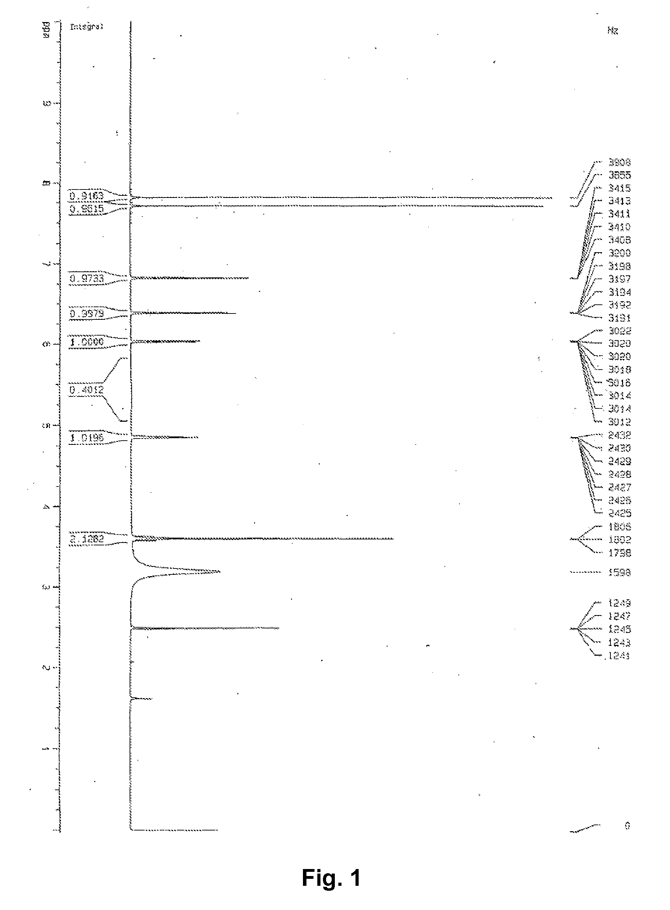

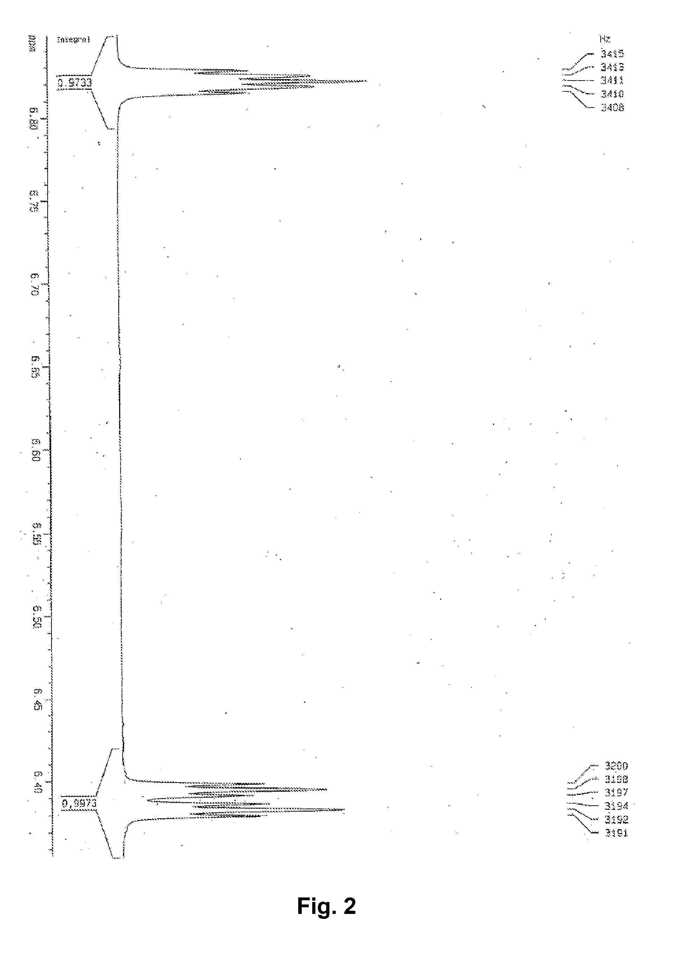

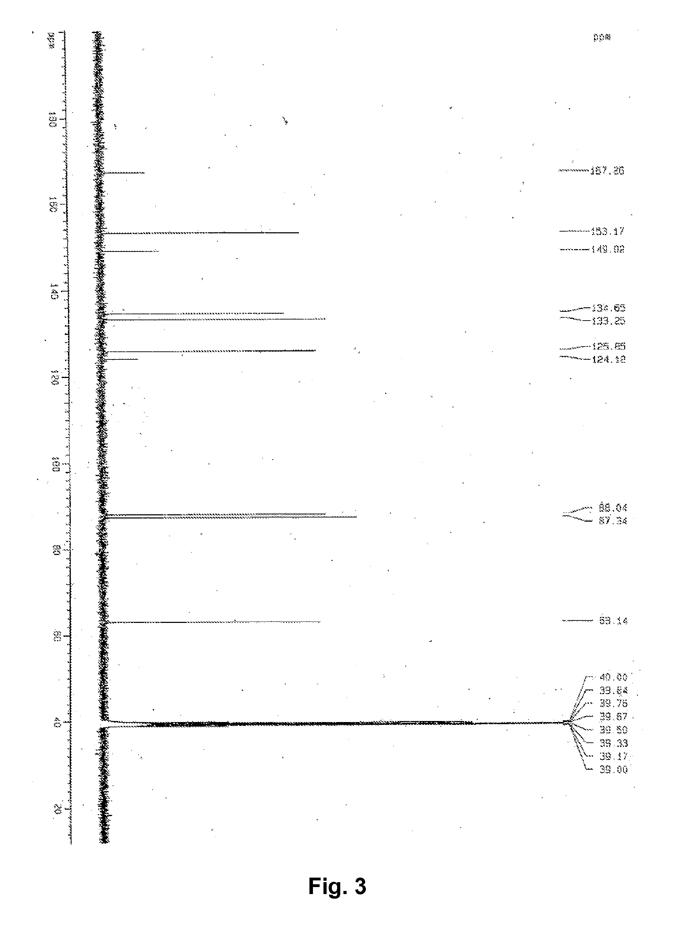

Fig.1, 2, and 3 illustrate NMR spectra of the product obtained in Example 3.

Example 1

Preparation of DDI from 5'-acetoxy-D4l

20 g of 5'-acetoxy-D4l was dissolved in 200 mL of acetonitrile. The solution was cooled to 5°C. Aqueous NaOH was added dropwise to the reaction mixture while stirring and the pH of the solution was maintained above 8.5. The completion of the protecting group removal was followed by TLC. After deprotection, 70 g of Raney nickel was added and the intermediate was hydrogenated with hydrogen gas at room temperature and normal pressure. The completion of hydrogenation was determined by HPLC. The solution was condensed under reduced pressure, and then neutralized with acetic acid. The crystalline product was filtered and dried. About 14 g of a solid was obtained. The dried DDI was recrystallized from methanol to obtain high purity DDI.

Example 2

Preparation of DDI from 2',3'-Didehydro-5'-O-(2,5,5-trimethyl-1,3-dioxolan-4-on-2-yl)-2',3'-dideoxyinosine

20 g of 2',3'-Didehydro-5'-O-(2,5,5-trimethyl-1,3-dioxolan-4-on-2-yl)-2',3'-dideoxyinosine was dissolved in 200 mL of acetonitrile. The solution was cooled to 5°C. Aqueous NaOH was slowly added dropwise into the reaction mixture while stirring. The pH of the solution was maintained at above 8.5. The completion of deprotection was followed by TLC. After completion, 50 g of Raney Nickel was added and the intermediate was hydrogenated with hydrogen gas at room temperature and normal pressure. The completion of hydrogenation was followed by HPLC. The solution was condensed under reduced pressure, and then neutralized with acetic acid. The crystallized product was filtered and dried. About 9 g of solid was obtained. The dried DDI was recrystallized with methanol to obtain high purity DDI.

Example 3

Preparation of Na-D4l·2H₂O

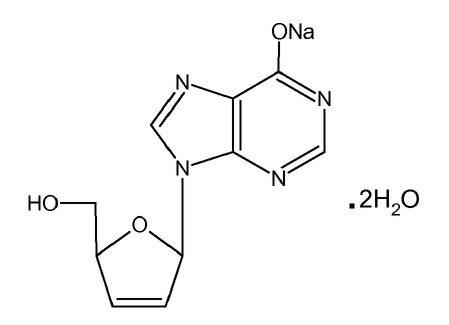

45 mL of 1 N NaOH solution was cooled down to below 10°C. Then 10 g of D4l was added, and the reaction mixture was stirred for 0.5 hours while maintaining the temperature at 10-15°C. The pH of the solution was maintained at between 9.5 and 11. Then, 600 mL of isopropanol was added slowly, and stirred for 2 hours at room temperature until the product crystallized. The mixture was filtered and the crystals were washed with 100 mL of isopropanol, and then dried in vacuum for 24 hours at a temperature of below 50°C. As a result, about 11 g of Na-D4l·2H₂O was obtained.

Identification tests:

(1) NMR: See Figs.1-3.

(2) Water (Karl Fischer titration): theoretical value is 12.3% and the test result was 12.4%.

In the above methods, NaOH can be replaced by KOH or strong organic base to obtain a corresponding salt of D4l.

Example 4

Preparation of DDI from purified X-D4l·nH₂O

100 g of Na-D4l·2H₂O was dissolved in 1000 mL of methanol. The reaction vessel was flushed with nitrogen. 500 g of activated Raney nickel was added. The reaction mixture was placed under an atmosphere of hydrogen gas for 10 hours under normal pressure. The completion of the reaction was followed by HPLC. At the completion of the reaction, the solution was filtered and condensed under reduced pressure to a volume of 100 mL. The condensed solution was then cooled to room temperature, and acetic acid was added dropwise while stirring. The pH was adjusted to between 6.5 and 7 and a white solid crystallized out. The mixture was stirred for 1 hour at room temperature and filtered. The filter cake was washed with 100 mL of methanol and dried at a temperature of between 55°C and 60°C. About 60 g of DDI was obtained. If needed, a high purity product can be obtained by recrystallization from methanol.

## Claims

1. A method of preparing compound I, comprising:
(a) removing the protecting group in position 5' in compound II by hydrolysis under basic condition, and simultaneously enolizing of the carbonyl group of the purine ring to yield a stable salt of of 2',3'-didehydro-2',3'- dideoxyinosine having formula III,
(b) producing the salt of 2',3'-dideoxyinosine of formula IV via catalytic hydrogenation, and
(c) neutralizing the salt of 2',3'-dideoxyinosine of formula IV with acid to obtain didanosine having formula I,
which is illustrated by the following chemical equations: 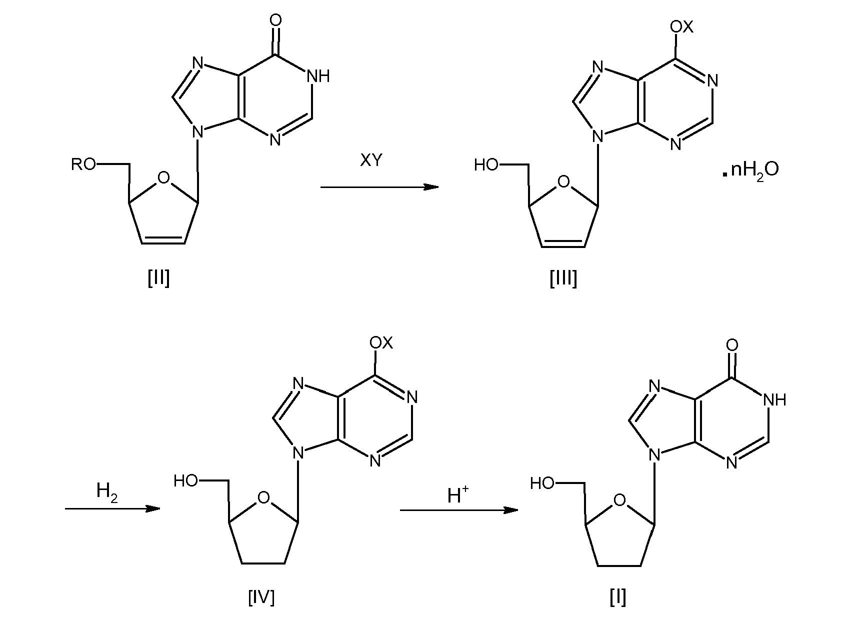
wherein R is acyl, alkyl or silyl; X is a cation; Y is a corresponding anion; and n is between 0 and 3.

2. The method of claim 1, wherein X is an alkali metal ion, an ammonium ion, a quaternary ammonium cation, such as, Na⁺, K⁺, NH₄⁺ or CH₃NH₃⁺.

3. The method of claim 1, wherein the solvent used for the preparation of compound IV from compound III through catalytic hydrogenation is selected from water, ammonia water, lye, an alcohol, a mixture of alcohol and water, a mixture of a ketone and water, a mixture of a nitrile and water, etc., e.g., methanol, a mixture of methanol and water, a mixture of ethanol and water, a mixture of isopropanol and water, a mixture of acetonitrile and water, a mixture of acetone and water, ammonia water or a mixture of methylamine and water; the catalyst used in the hydrogenation is Raney nickel or palladium on carbon, and the pH during the catalytic hydrogenation is maintained at between 8 and 14.

4. The method of claim 1, wherein the acid used for the preparation of compound I from compound IV through neutralization is an inorganic acid or an organic acid, such as, e.g., hydrochloric acid or acetic acid.

5. The method of claim 1, wherein an intermediate in the synthesis of the compound of formula I is the salt of 2',3'-didehydro-2',3'- dideoxyinosine, which has the structure of formula III, as follows: 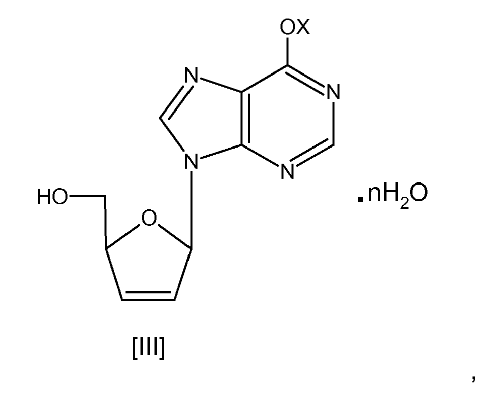 X is an alkali metal ion, an ammonium ion, or a quaternary ammonium cation, such as, Na⁺, K⁺, NH₄⁺ or CH₃NH₃⁺, and n is between 0.5- and 3.

6. The method of claim 5, wherein said salt of 2',3'-didehydro-2',3'-dideoxyinosine of formula III is obtained by hydrolysis of compound II in a solution having a pH of between 8.5 and 14 or a pH greater than 8.5, followed by crystallization from an organic solvent; and the structural formula of compound II is as follows: 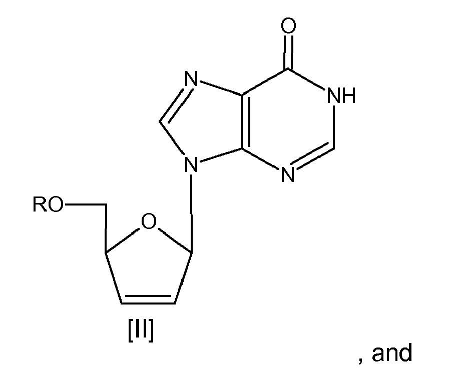 R is an acyl, an acetoxy, an alkyl or a silyl, such as acetyloxy, 2,5,5-trimethyl-1,3-dioxolane -4-carboxylate, or tert-butyldimethylsilyl.

7. The method of claims 1 or 4, wherein the new compound of formula III or its hydrate can be used in subsequent steps after purification or without purification in a crude form.

8. The method of claim 5, wherein the base used in the hydrolysis for the preparation of salt of 2',3'-didehydro-2',3'- dideoxyinosine is an inorganic base, an organic base, or a mixture thereof, such as, e.g., potassium hydroxide, sodium hydroxide, potassium carbonate, sodium carbonate, or methylamine, or a mixture thereof; said organic solvent is an alcoholic solvent, a ketonic solvent, or a nitrilic solvent, such as isopropanol, acetone, acetonitrile, or tetrahydrofuran.
